# EUROPEAN PATENT SPECIFICATION

(11) **EP 0 330 892 B1**
(45) Date of publication and mention of the grant of the patent: **06.07.1994**
(21) Application number: 89102407.7
(22) Date of filing: 11.02.1989
(51) Int. Cl.: A61M 1/14

(54) **Dialysis system**
Dialysesystem
Système de dialyse

(30) Priority: 03.03.1988 SE 8800757
(43) Date of publication of application: 06.09.1989
(60) Divisional application: 93100634.0
(73) Proprietor: GAMBRO AB, S-220 10 Lund (SE)
(72) Inventor: Sternby, Jan Peter, Rochester Minn. 55902 (US)
(74) Representative: Boberg, Nils Gunnar Erik

(56) References cited:
- EP-A- 0 097 366
- EP-A- 0 122 604
- EP-A- 0 204 174
- DE-A- 2 825 134

## Description

### TECHNICAL FIELD

The following invention relates to a dialysis system, comprising means for the conducting of blood and dialysis liquid respectively on either side of one or more membranes in a dialyser, means for the control of one or more parameters of the dialysis liquid upstream of the dialyser and means for measuring at least one parameter of the dialysis liquid downstream of the dialyser.

The term dialysis system here also comprises systems for similar treatments, e g haemodiafiltration, that is to say dialysis with such high ultrafiltration, that certain quantity of replacement liquid has to be supplied to the patient to make up for the filtrate removed.

### BACKGROUND ART

In a normal dialysis treatment there is normally no feedback of patient's data to the dialysis machine. Thus the patient's temperature, blood values etc may be no matter how abnormal without the machine reacting, as long as the machine's own values are correct.

As an example of systems which control the composition of the dialysis liquid irrespectively of the blood parameters of the individual patient can be mentioned, for example, those described in the US patents 4 158 034, 4 293 409 and 4 508 622.

Of special interest is the last mentioned US patent and corresponding European publication EP-A-0 097 366, disclosing a dialysis system in which one or more parameters are measured upstream and downstream a dialyzer, whereafter the measured values are compared with each other. Such a comparison is, however, difficult to perform.

The system in accordance with the invention may be said also to constitute a further development of the dialysis system developed by the Gambro Group which is marketed under the name of Gambro AK-10 and Gambro AK-100. Details of this system are described more fully, for example, in the two first mentioned US patents and in the US patents 4 194 974, 4 191 359, 4 585 552 and 4 536 201 and the European patent publications EP B 22 922, EP A 204 260, EP A 204 174, EP A 208 090 and EP A 238 809.

### DISCLOSURE OF INVENTION

It is an object of the present invention to increase the patient's comfort by making the treatment more individualized. This can be done in that certain patient's parameters are measured and adapted to control the dialysis machine used. Examples of such patient's parameters may be blood pressure, temperature, blood gas content, electrolyte content, pH-status etc. Such measurements should be made wholly without blood contact, so that transmitters etc need not be of the disposable type or be sterilized between each treatment.

The dialysis system in accordance with the invention is characterized in the first place by means for a comparison of the parameter measured downstream of the dialyser with the set value of the corresponding parameter upstream of the dialyser. Through such a comparison a measure of the dialysis performed is obtained, and with the help of this measure, and knowledge of other, e g adjusted values relevant for the analysis, conditions on the blood side can be determined theoretically and thereby controlled.

The comparative value measured can be used simply for a control of the composition of the dialysis liquid an thereby an indirect passive control of the composition of the blood. Preferably though the dialysis sytems in accordance with the invention is provided instead with means for using the result measured for the calculation of a blood parameter which is altered as a function of the parameter measured, for the purpose of calculation and/or control of this blood parameter in the patient.

Inasfar as the electrolyte composition of the blood is concerned, it has been found that it is a prerequisite to obtain a value of this by measuring the conductivity of the dialysis liquid which passes the dialyser. Through the influence of the blood the conductivity of the incoming dialysis liquid will be altered and the magnitude of the alteration will depend on the composition of the blood. Since the conductivity for the most part is produced by sodium ions together with its anions, the conductivity largely becomes a measure of the sodium content in the dialysis liquid and in the blood. Preferably, therefore, the dialysis system in accordance with the invention is provided with a conductivity meter arranged downstream of the dialyser for measuring the conductivity in the dialysis liquid for the purpose of calculation and/or control of the conductivity and/or the sodium content in the blood.

Alternatively the dialysis system in accordance with the invention can be provided with one or more ion-selective electrodes for measuring the content of one or more defined ions in the dialysis liquid for the purpose of calculation and/or control of the corresponding ion content in the blood. In this way other ions beside sodium can also be measured, such as for example calcium and potassium.

It will be obvious to those versed in the art, that many other blood parameters too can be calculated and/or controlled with the help of corresponding values measured on the dialysis liquid side. Thus it should be possible, for example, to provide the dialysis system in accordance with the invention with a gas analyser arranged downstream of the dialyser for measuring the gas content in the dialysis liquid for the purpose of calculation and/or control of corresponding blood gases. However, this has not yet been verified in practice.

It will be obvious, moreover, to those versed in the art that it would be better to measure directly various ions with the help of ion-selective transmitters instead of taking the route via a conductivity meter. With the technique available today, however, a conductivity measurement is appreciably more stable, more accurate, simpler and less expensive. Preferably, therefore, just one conductivity meter is used. It is intended then with the help of this to measure the conductivity on the dialysis liquid side so a to calculate thereafter theoretically the conductivity on the blood side. On the assumption that the conductivity largely depends upon the sodium content, the sodium passage or sodium dialysance will be determining for the effect of the blood side on the conductivity value of the dialysis liquid measured downstream of the dialyser. A simple mass balance for sodium at constant flow and assuming the so-called Donnan effect, furnishes the formula:

$\text{Cdout = Cdin + (Cbin - Cdin) xK}$

wherein
Cdout = the conductivity or the concentration of sodium in used dialysis liquid.
Cdin = the conductivity or the concentration of sodium in fresh dialysis liquid.
Cbin = the conductivity or the concentration of sodium in untreated blood.
K = proportionality constant depending on the flow.
If the ultrafiltration is kept at zero, K becomes D1/Qd (=relative dialysance) where
D1 = dialysance value for sodium/conductivity.
Qd = dialysis liquid flow.
The same formula applies also where the ultrafiltration is different from zero, but with a modified value of K.

When the factor K is known, and by measuring Cdout, it is possible to calculate Cbin, if at the same time Cdin is put to equal the set value of the corresponding matter.

The factor K can be determined in two different ways. The simplest is, starting out from a knowledge of dialyser, blood flow and liquid flow, to determine it empirically. It is also possible to determine K continuously by a regular change of the set conductivity of the liquid. At each change a measure of K is obtained from a comparison between the conductivity measured upstream and downstream of the dialyser respectively. This method requires a liquid conductivity which during the treatment is altered by steps, e g every 6th minute by 0.5 mS/cm, up or down respectively from the set value, which ought to be in the order of magnitude 13.7 - 14.0 mS/cm. These changes are so rapid, that the body quite likely would not keep up with them to any appreciable extent. At the same time they are so small that they do not notably affect the aforementioned formula.

The dialysis system in accordance with the invention appropriately is provided with means for the control of the composition of the dialysis liquid upstream of the dialyser as a function of the parameter measured in such a manner that the dialysis result becomes a direct function of the corresponding blood parameter. As a result, conditions on the blood side will be directly determining for the dialysis and not the preset values on the dialysis liquid side, as has been the case normally up to now. If the dialysis system in accordance with the invention is adapted for determination of the conductivity and/or the sodium content in the blood, the said means for adjusting of the composition of the dialysis liquid upstream of the dialyser may be adapted to bring about an equilibrium between the conductivities in the dialysis liquid and in the blood. In this manner a conductivity adapted to the individual is obtained, which should provide maximum comfort for the patient.

The dialysis system in accordance with the invention may additionally comprise, in a manner known in itself, means for measuring the actual dialysis liquid parameter, which is measured downstream of the dialyser, also just upstream of the same. The system suitably is provided also with a shunt line for conducting the fresh dialysis liquid past the dialyser directly to the measuring device normally placed downstream of the dialyser, for the purpose of calibration of the measuring devices used. It should be noted in this connection that any minor fault common to both measuring devices is of less importance for the final result than if only one of the measuring devices were to present an incorrect measuring value.

The dialysis system in accordance with the invention, in a manner known in itself, may comprise means for the measuring of the said parameter of the dialysis liquid both upstream and downstream of the dialyser. In such case the parameter measured after the dialyser instead may be compared with the value of the same parameter measured upstream of the dialyser. In practice it has been found simpler, however, to use a corresponding set value for comparison. This set value, of course, has already been entered into the system.

### BRIEF DESCRIPTION OF DRAWING

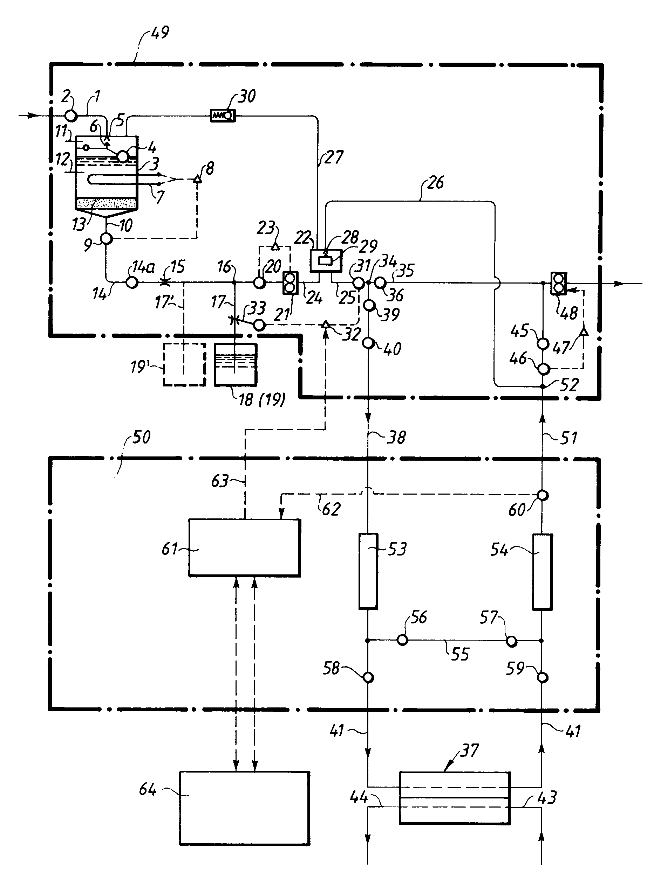

The invention is described in more detail in the following with reference to the attached drawing which shows a block diagram comprising a mixing module, a flow control module, an external computer and a dialyser.

### BEST MODE OF CARRYING OUT THE INVENTION

On the drawing an inlet duct for fresh water is designated 1. This duct 1, which is provided with an inlet valve 2, leads to a water vessel 3. This vessel 3 is provided with a float valve 4 which is adapted to close the water intake with the help of a closing cone 6 when the water vessel has been filled. The water vessel, moreover, comprises means 7 for the heating of the water, e g in the form of a heating loop. This heating loop is controlled by a temperature regulating means 8 which in turn is controlled by a temperature transmitter 9 in the outlet duct 10 from the vessel 3. Furthermore the vessel 3 comprises maximum and minimum monitors 11 and 12 respectively, shown schematically, for sensing the liquid level in the vessel. These level monitors may be adapted, for example, to control directly the inlet valve 2. Finally the vessel contains a filter element 13, shown schematically, which in the first place is intended to remove any solid particles from the water, but which in practice also removes a certain amount of gas, e g free gas bubbles.

After heating the fresh water supplied is conducted via the outlet line 10, the temperature transmitter 9, a duct 14 with a shut-off valve 14a, and a throttle valve 15 to a branching point 16. Here a branch 17 is connected which normally starts out from a source 18 of salt solution concentrate. Generally this source quite simply consists of a drum with a salt solution. When the system shown is to be sterilized, however, this drum is replaced by one containing a sterilizing agent which is marked (19) in the figure. In practice this is the simplest solution. It would also be possible of course, would this be required for any special reason, to connect a further line 17′ to the system parallel with the line 17 for the connection of a separate vessel 19′. This has been indicated in the figure by broken lines.

From the point 16 the liquid flows via pressure gauge 20 and a pump 21 to a bubble separator 22. The pressure gauge 20 here controls the pump via a pressure regulating means 23. The supply line to the bubble separator 22 has been designated 24.

From the bubble separator 22 starts out beside the ordinary line 25, also a line 26 for the removal of the separated gas, and also a return line 27. The inlet 28 to the line 26 is controlled by a float valve 29 which closes this inlet when the bubble separator 22 is filled with liquid in connection with sterilization. The return line 27 is provided with a spring-loaded check valve 30 and leads back to the liquid vessel 3.

The line 25 leads to a conductivity meter 31 which via a control means 32 controls a throttle valve 33 in the line 17 for salt solution concentrate. Alternatively it may control instead a concentrate pump in the same line 17. After the conductivity meter 31 the liquid flow reaches a new branching point 34 from which originates shunt line 35 with a valve 36. This shunt line 35 is used when it is desired to couple the liquid flow past the dialyser, e g when an abnormality is detected in the dialysis liquid, for example, of its temperature or salt content. Normally the liquid otherwise flows to the dialyser 37 via a line 38 which contains a valve 39 and a flow meter 40. On its way to the dialyse 37 the dialysis liquid passes a flow control module which, as a whole, is designated 50. The components described up to now, on the other hand, are included in a liquid preparation module which, as a whole, is designated 49. On its way from the dialyser 37 the dialysis liquid once agains passes the flow control module 50 to be returned via a line 51 to the module 49. At a point 52 the line 51 is coupled together with the line 26 from the bubble separator 22.

The lines or tubings which are normally coupled to the dialyser 37 are designated 41. During sterilization, though, these tubings are coupled to a "safety-bypass", not shown in the drawing. An example of such a "safety-bypass" is described in the US patent 4 122 010 and it need not be described in greater detail, therefore, in connection with the present invention. The inlet and outlet respectively on the blood side of the dialyser are designated 43 and 44.

A blood detector 45 provided in the module 49 raises an alarm which possibly shuts down the whole system if blood is detected in the dialysis liquid. This blood detector, for example, may be a transparent tubing opposite an otherwise shielded photocell device which directly senses the occurrence of any blood in the dialysis liquid. Before the blood detector a pressure gauge 46 is passed which via a control means 47 controls a liquid pump 48. Thereafter the liquid flow is conducted finally to a drain, not shown.

In the module 50 the dialysis liquid flow to the dialyser is measured in a first measuring device 53. In the same manner the dialysis liquid flow from the dialyser is measured in a second measuring device 54. Through a comparison of the values obtained the ultrafiltration in the dialyser can be determined. The two measuring devices 53 and 54 may be included, for example, in a differential measuring arrangement of the type which is described in British patent 2 003 274 or in the US patent 4 585 552. During the calibration of the measuring devices the dialyser is shunted with the help of a bypass line 55 comprising two valves 56 and 57. At the same time the lines to and from the dialyser are shut off with the help of valves 58 and 59 respectively.

After the dialysis liquid flow has passed the measuring device 54 it passes a device 60 for the measurement of at least one parameter representative for it. In the example shown this device consists of a conductivity meter. The value measured is passed to a microprocessor 61, which is indicated by the broken line 62. Here the measured value is compared with the set value of the same parameter before the dialyser, that is to say in this case the conductivity. This set value is adjusted with the help of the microprocessor 61 which is indicated by the broken line 63. This takes place thus with the help of the aforementioned control means 32. If desired the microprocessor 61 may be provided furthermore with data from or it may furnish data to a further personal computer 64. With the help of this further computer, for example, the individual values of the patient can be transferred to the microprocessor 61 at the same time as the values calculated for the blood side of the dialyser can be noted.

Naturally the invention is not limited solely to the embodiment described above but can be varied within the framework of the subsequent claims. For example, the system in accordance with the example of an application described above is based on only one concentrate, that is to say that taken from the container 18. The invention also may be applied, however, e g when two concentrates are used in the manner which is described in EP-B1-22922. The value measured in the device 60 is compared in such a case with the ready-mixed dialysis liquid, that is to say after mixing in of the two concentrates used. The two concentates, for the rest, need not be in liquid form. Concentrate in powder form may also be used e g in accordance with what is described in European patent application 87.118987.4 (EP-A-278 100).

## Claims

1. A dialysis system comprising means (41,41,43,44) for the conducting of dialysis liquid and blood respectively on either side of one or more membranes in a dialyser (37), means (61) for controlling one or more parameters of the dialysis liquid upstream of the dialyser (37) and means (60) for measuring at least one parameter of the dialysis liquid downstream of the dialyser, **characterized** by means (61) for a comparison of the parameter measured downstream of the dialyser (37) with the set value of the corresponding parameter upstream of the dialyser (37).

2. A dialysis system in accordance with claim 1, **characterized** by means (61) for using the result measured for the calculation of a blood parameter, which is altered as a function of the parameter measured, for the purpose of calculation and/or control of this blood parameter.

3. A dialysis system in accordance with claim 1 or 2, **characterized** by a conductivity meter (60) arranged downstream of the dialyser (37) for the measurement of the conductivity in the dialysis liquid for the purpose of calculation and/or control of the conductivity and/or the sodium content in the blood.

4. A dialysis system in accordance with claim 1 or 2, **characterized** by an ion-selective electrode (60) for the measurement of the content of a certain ion in the dialysis liquid for the purpose of calculation and/or control of the corresponding ion content in the blood.

5. A dialysis system in accordance with claim 1 or 2, **characterized** by a gas analyser (60) arranged downstream of the dialyser (37) for the measurement of the gas content in the dialysis liquid for the purpose of calculation and/or control of corresponding blood gases.

6. A dialysis system in accordance with claim 3, **characterized** in that the conductivity and/or the sodium conetnet are calculated according to the formula
$\text{Cdout = Cdin + (Cbin - Cdin) xK}$
wherein
Cdout = the conductivity or the concentration of sodium in the dialysis liquid used.
Cdin = the conductivity or the concentration of sodium in fresh dialysis liquid.
Cbin = the conductivity or the concentration of sodium in untreated blood.
K = proportionality constant depending on the flow.

7. A dialysis system in accordance with claim 6, **characterized** in that the factor D1/Qd is determined empirically based on knowledge of the dialyser, blood flow, liquid flow etc used.

8. A dialysis system in accordance with claim 6, **characterized** in that the factor K is determined through a preferably regular change of the set conductivity of the liquid, e g +/-0.5 mS/cm from an initial value of in the order of magnitude of 13.7 - 14.0 mS/cm.

9. A dialysis system in accordance with anyone of the preceding claims, **characterized** by means (61,32) for the control of the composition of the dialysis liquid upstream of the dialyser (37) as a function of the parameter measured in such a manner, that the dialysis result becomes a direct function of the corresponding blood parameter.

10. A dialysis system in accordance with claim 9, adapted for the determination of the conductivity and/or the sodium content in the blood, **characterized** in that the said means (61,32) for the adjustment of the composition of the dialysis liquid upstream of the dialyser are adapted to bring about an equilibrium between the conductivities in the dialysis liquid and in the blood respectively.

11. A dialysis system in accordance with anyone of the preceding claims comprising means (31) for the measuring of the same dialysis liquid parameter which are measured downstream of the dialyser (37) also just upstream of the same, **characterized** by a shunt line (55) for conducting the fresh dialysis liquid past the dialyser (37) directly to the measuring device (60) normally placed after the dialyser for the purpose of calibration of the measuring devices (31,60) used.

## Patentansprüche

1. Ein Dialysesystem mit Mitteln (41,42,43,44) zum Leiten von Dialyseflüssigkeit bzw. Blut auf jeder Seite einer oder mehrerer Membranen in einem Dialysator (37), Mitteln (61) für die Steuerung eines oder mehrerer Parameter der Dialyseflüssigkeit aufstromig vom Dialysator (37) und Mitteln (60) zum Messen mindestens eines Parametern der Dialyseflüssigkeit abstromig vom Dialysator, gekennzeichnet durch Mittel (61) für einen Vergleich des abstromig vom Dialysator (37) gemessenen Parameters mit dem festgesetzten Wert des entsprechenden Parameters aufstromig vom Dialysator (37).

2. Ein Dialysesystem nach Anspruch 1, gekennzeichnet durch Mittel (61), um des gemessene Resultat für die Berechnung eines Blutparameters zu nutzen, der als eine Funktion des gemessenen Parameters zum Zweck der Berechnung und/oder Steuerung dieses Blutparameters geändert wird.

3. Ein Dialysesystem nach Anspruch 1 oder 2, gekennzeichnet durch einen Leitfähigkeitsmesser (60), der abstromig vom Dialysator (37) für die Messung der Leitfähigkeit in der Dialyseflüssigkeit zum Zweck der Berechnung und/oder Kontrolle der Leitfähigkeit und/oder des Natriumgehaltes in dem Blut angeordnet ist.

4. Ein Dialysesystem nach Anspruch 1 oder 2, gekennzeichnet durch eine Ionentrennelektrode (60), für die Messung des Gehaltes eines bestimmten Ions in der Dialyseflüssigkeit zum Zweck der Berechnung und/oder Kontrolle des entsprechenden Ionengehaltes in dem Blut.

5. Ein Dialysesystem nach Anspruch 1 oder 2, gekennzeichnet durch einen Gasanalysator (60), der abstromig vom Dialysator (37) für die Messung des Gasgehaltes in der Dialyseflüssigkeit zum Zweck der Berechnung und/oder Kontrolle entsprechender Blutgase angeordnet ist.

6. Ein Dialysesystem nach Anspruch 3, dadurch gekennzeichnet, daß die Leitfähigkeit und/oder der Natriumgehalt nach der Formel berechnet werden
$\text{Cdout = Cdin + (Cbin - Cdin) x K}$
worin
Cdout = die Leitfähigkeit und die Natriumkonzentration in der benutzten Dialyseflüssigkeit.
Cdin = die Leitfähigkeit oder die Natriumkonzentration in frischer Dialyseflüssigkeit.
Cbin = die Leitfähigkeit oder die Konzentration von Natrium in unbehandeltem Blut.
K = Die Proportionalitätskonstante, die von dem Fluß abhängig ist.

7. Ein Dialysesystem nach Anspruch 6, dadurch gekennzeichnet, daß der Faktor D1/Qd empirisch bestimmt wird, basierend auf der Kenntnis des benutzten Dialysators, Blutflusses, Flüssigkeitsstromes usw..

8. Ein Dialysesystem nach Anspruch 6, dadurch gekennzeichnet, daß der Faktor K durch ein vorzugsweise regelmäßiges Verändern der festgesetzten Leitfähigkeit der Flüssigkeit bestimmt wird, zum Beispiel +/- 0.5mS/cm von einem Anfangswert aus in der Größenordnung von 13.7 - 14.0 mS/cm.

9. Ein Dialysesys6tem nach einem der vorangehenden Ansprüche, gekennzeichnet durch Mittel (61,32) für die Kontrolle der Zusammensetzung der Dialyseflüssigkeit aufstromig vom Dialysator (37) als eine Funktion des in der Art gemessenen Parametern, daß des Dialyseresultat eine direkte Funkton des entsprechenden Blutparameters wird.

10. Ein Dialysesystem nach Anspruch 9, geeignet für die Bestimmung der Leitfähigkeit und/oder des Natriumgehaltes in dem Blut, dadurch gekennzeichnet, daß die genannten Mittel (61,32) für die Einstellung der Zusammensetzung der Dialyseflüssigkeit aufstromig vom Dialysator geeignet sind, ungefähr ein Gleichgewicht zwischen den Leitfähigkeiten in der Dialyseflüssigkeit bzw. in dem Blut herzustellen.

11. Ein Dialysesystem nach einem der vorangehenden Ansprüche mit Mitteln (31) für das Messen desselben Dialyseflüssigkeitsparameters, der abstromig vom Dialysator (37) gemessen wird, auch genau aufstromig von demselben, gekennzeichnet durch eine Shuntleitung (55) für das Leiten der frischen Dialyseflüssigkeit vorbei an dem Dialysator (37) direkt zu der Meßvorrichtung in (60), die normalerweise nach dem Dialysator zum Zweck der Kalibrierung der benutzten Meßvorrichtungen (31,60) angeornet ist.

## Revendications

1. Système de dialyse comprenant des moyens (41,41,43,44) pour la circulation de liquide de dialyse et de sang respectivement sur chaque face d'une ou plusieurs membranes dans un dialyseur (37), des moyens (61) de réglage d'un ou plusieurs paramètres du liquide de dialyse en amont du dialyseur (37) et des moyens (60) de mesure d'au moins un paramètre du liquide de dialyse en aval du dialyseur, caractérisé en ce qu'il comprend des moyens (61) de comparaison du paramètre mesuré en aval du dialyseur (37) avec la valeur de consigne du paramètre correspondant en amont du dialyseur (37).

2. Système de dialyse suivant la revendication 1, caractérisé en ce qu'il comprend des moyens (61) pour utiliser le résultat mesuré pour le calcul d'un paramètre du sang, qui est modifié en fonction du paramètre mesuré, dans le but de calculer et/ou régler ce paramètre du sang.

3. Système de dialyse suivant la revendication 1 ou 2, caractérisé en ce qu'il comprend un conductivimètre (60) placé en aval du dialyseur (37) pour mesurer la conductivité dans le liquide de dialyse, dans le but de calculer et/ou régler la conductivité et/ou la teneur en sodium du sang.

4. Système de dialyse suivant la revendication 1 ou 2, caractérisé en ce qu'il comprend une électrode à sélectivité ionique (60) pour la mesure de la teneur en un certain ion dans le liquide de dialyse, dans le but de calculer et/ou régler la teneur en ion correspondant dans le sang.

5. Système de dialyse suivant la revendication 1 ou 2, caractérisé en ce qu'il comprend un analyseur de gaz (60) placé en aval du dialyseur (37) pour la mesure de la teneur en gaz du liquide de dialyse, dans le but de calculer et/ou régler les gaz correspondants du sang.

6. Système de dialyse suivant la revendication 3, caractérisé en ce que la conductivité et/ou la teneur en sodium sont calculées conformément à la formule
$\text{Cdout = Cdin + (Cbin- Cdin) x K}$
dans laquelle
Cdout = conductivité ou concentration en sodium dans le liquide de dialyse usé,
Cdin = conductivité ou concentration en sodium du liquide de dialyse neuf,
Cbin = conductivité ou concentration en sodium du sang non traité,
K = constante de proportionnalité dépendant du débit.

7. Système de dialyse suivant la revendication 6, caractérisé en ce que le facteur D1/Qd est déterminé empiriquement sur la base de la connaissance du dialyseur, du débit de sang, du débit de liquide, etc., utilisés.

8. Système de dialyse suivant la revendication 6, caractérisé en ce que le facteur K est déterminé par l'intermédiaire d'une variation de préférence régulière de la conductivité de consigne du liquide, par exemple de ± 0,5 mS/cm à partir d'une valeur initiale de l'ordre de 13,7-14,0 mS/cm.

9. Système de dialyse suivant une quelconque des revendications précédentes, caractérisé en ce qu'il comprend des moyens (61,32) pour le réglage de la composition du liquide de dialyse en amont du dialyseur (37) en fonction du paramètre mesuré, d'une manière telle que le résultat de la dialyse devient une fonction directe du paramètre correspondant du sang.

10. Système de dialyse suivant la revendication 9, adapté à la détermination de la conductivité et/ou de la teneur en sodium du sang, caractérisé en ce que les dits moyens (61,32) pour le réglage de la composition du liquide de dialyse en amont du dialyseur sont prévus pour engendrer un équilibre entre les conductivités du liquide de dialyse et du sang respectivement.

11. Système de dialyse suivant une quelconque des revendications précédentes, comprenant des moyens (31) pour la mesure du même paramètre de liquide de dialyse qui est mesuré en aval du dialyseur (37), également juste en amont de ce dernier, caractérisé en ce qu'il comprend un conduit de contournement (55) pour amener le liquide de dialyse neuf au-delà du dialyseur (37) directement au dispositif de mesure (60) normalement placé après le dialyseur, dans le but d'étalonner les dispositifs de mesure (31,60) utilisés.
